# EUROPEAN PATENT APPLICATION

(11) **EP 3 042 638 A1**
(43) Date of publication of application: **13.07.2016**
(21) Application number: 15190225.1
(22) Date of filing: 16.10.2015
(51) Int. Cl.: A61F 2/90

(54) **METHOD OF MANUFACTURING ANTI-MIGRATION STENT AND STENT MANUFACTURED THEREBY**

(30) Priority: 21.10.2014 KR 20140142391
(71) Applicant: Taewoong Medical Co., Ltd., Gyeonggi-do 10022 (KR); Shin, Kyong-Min, Gyeonggi-do 12548 (KR)
(72) Inventor: SHIN, Kyong-Min, 12548 Gyeonggi-do (KR); HONG, Young ill, 10056 Gimpo-si (KR)
(74) Representative: McCartney, Jonathan William

(57) **Abstract**

The present invention relates to a method of manufacturing an anti-migration stent and an anti-migration stent manufactured thereby and, more particularly, to an anti-migration stent including a hollow stent body formed by interweaving a superelastic shape memory alloy wire diagonally such that upper and lower wires alternately cross over and under each other, forming multiple diamond-shaped spaces. After placing and holding a rod underneath the wire of the hollow stent body, and heating the stent body, an anti-migration bent part is formed on the wire, protruding vertically to form an outline corresponding to a cross-sectional shape of the rod, which makes stent production easy. The stent is prevented from migrating in a lumen of a human body by the bent part protruding vertically and diagonally from the stent body, and easy to insert into and remove from the lumen, preventing the conventional problem damaging an inner surface of the lumen.

## Description

### CROSS REFERENCE TO RELATED APPLICATION

The present application claims priority to Korean Patent Application No. 10-2014-0142391, filed October 21, 2014, the entire contents of which is incorporated herein for all purposes by this reference.

### BACKGROUND OF THE INVENTION

### Field of the Invention

The present invention relates to a method of manufacturing an anti-migration stent and an anti-migration stent manufactured thereby. More particularly, the present invention relates to a method of manufacturing an anti-migration stent and an anti-migration stent manufactured thereby wherein superelastic shape memory alloy wires are interwoven diagonally in opposite directions to cross each other, thereby forming a hollow stent body, and anti-migration bent parts are formed on the hollow stent body in such a manner that the wires are partially bent to protrude upward vertically from the outer circumference of the hollow stent body, so that the stent is less likely to migrate from an inner surface of a lumen of a human body, and insertion and removal of the stent are easy, thereby solving conventional problems of pricking and damaging the inner surface of the lumen during the insertion and removal of the stent.

### Description of the Related Art

Generally, when stenosis occurs due to tumors or other reasons in lumens of the human body, including digestive organs such as the duodenum, the biliary tract, and the esophagus, urinary organs such as the urethra, and respiratory organs such as the trachea, the human body cannot perform its normal functions. Accordingly, a stent is inserted into the stenotic portion in a lumen of a human body to expand the stenotic portion so that the human body can function normally.

The stent includes a hollow stent body formed by interweaving at least one superelastic shape memory alloy wire diagonally such that the wire alternately crosses over and under itself to form a plurality of diamond-shaped spaces in the hollow stent body, so that the hollow stent body applies tension to inner and outer side of a lumen to expand the stenotic portion.

However, the conventional stent described above is composed of the hollow stent body alone, and has a problem in that the stent tends to move in a lumen of a human body to easily deviate from a lesion part into which the stent is implanted.

To address the problem of the conventional stent, various stents having an anti-migration structure have been presented.

First, as in patent documents 1 and 2, at least one of opposite ends of a hollow stent body has an expanded part in such a manner that the expanded part is slanted or has steps, so that the stent is brought into close contact with the inner surface of the lumen of the human body to prevent the migration thereof.

However, the stent has a limitation in that it is not able to completely realize the anti-migration effect because the stent cannot have an expanded part on at least one of opposite ends of the hollow stent body considering a manufacturing condition of the stent, and even if it is possible to manufacture an expanded part, the expanded part alone cannot ensure the stent to be securely held on the inner surface of a lumen of the human body.

Second, as in patent document 3, a stent is provided with an anti-migration bent part formed in such a manner that a wire is extended to slantingly protrude upward at multiple positions on an outer circumference of a hollow stent body, and thus the anti-migration bent part is held to an inner surface of a lumen in a human body, thereby preventing migration of the stent.

However, since it is difficult to produce an anti-migration bent part on the outer circumference of the hollow stent body, the stent has a problem in that production of the stent is inefficient, and it is not easy to operate successfully due to the growing volume of the stent from the use of an additional wire while the anti-migration bent part is produced. Though it is not problematic to insert the stent into a lumen of a human body since the anti-migration bent part slantingly protrudes in an opposite direction to which the stent is inserted into the lumen of the human body, when the stent is removed from the lumen, an end of the anti-migration bent part tends to bend back to prick an inner surface of the lumen in the human body. Accordingly, the stent has a problem in that it is difficult to remove from the lumen, and thus may lead to damage of the inner surface of the lumen in the human body upon removal.

### Documents of Related Art

(Patent Document 1) Korean Patent No. 10-0455359;
(Patent Document 2) Korean Patent Application Publication No. 10-2012-0004677; and
(Patent Document 3) Korean Patent No. 10-1171075.

### SUMMARY OF THE INVENTION

The invention is defined in the attached independent claims to which reference should now be made. Further, optional features may be found in the sub-claims appended thereto.

The present invention is intended to address the issues of a conventional stent in such a manner that superelastic shape memory alloy wire is diagonally interwoven such that the wire alternately crosses over and under itself, and an anti-migration bent part is provided on the hollow stent body by bending the wire such that the anti-migration bent part protrudes vertically and diagonally, thereby preventing migration of the stent in the lumen of the human body, and in such a manner that the stent may be easy to insert into and remove from the lumen, thereby preventing the conventional issue of pricking and damaging the inner surface thereof.

The present invention is also intended to provide excellent workability and productivity since the present invention is easily manufactured in such a manner that a hollow stent body is formed by interweaving superelastic shape memory alloy wires diagonally such that the wires alternately cross over and under each other, and that an anti-migration bent part is vertically protruded by placing and holding a rod underneath the wire of the hollow stent body and heating the stent body.

Accordingly, the present invention provides a method of manufacturing an anti-migration stent, the method including: producing a hollow stent body by interweaving at least one superelastic shape memory alloy wire diagonally such that the wire alternately crosses over and under itself to form a plurality of diamond-shaped spaces in the hollow stent body; placing and holding a rod underneath the wire of the hollow stent body; and heating the stent body to form an anti-migration bent part on the wire, the anti-migration bent part protruding vertically to form an outline corresponding to a cross-sectional shape of the rod.

The present invention also provides an anti-migration stent constituted to prevent migration in a lumen of a human body, the anti-migration stent including: a hollow stent body formed by interweaving at least one superelastic shape memory alloy wire diagonally in such a manner that upper and lower wires alternately cross over and under each other, thereby forming a plurality of diamond-shaped spaces in the hollow stent body, wherein an anti-migration bent part is provided on the hollow stent body by bending the wire such that the anti-migration bent part protrudes vertically and diagonally, thereby preventing migration of the stent in the lumen of the human body.

The anti-migration bent part of the present invention may be formed by bending the upper wire or the lower wire such that the upper wire or the lower wire protrudes upward vertically and diagonally from the hollow stent body.

The anti-migration bent part of the present invention may be configured in a semicircular or a polygonal structure.

The anti-migration stent of the present invention may further include a silicone reinforcement part formed on an inside of the anti-migration bent part through coating to increase tension strength of the anti-migration bent part.

Additionally, the present invention provides an anti-migration stent constituted to prevent migration in a lumen of a human body, the anti-migration stent including: a hollow stent body formed by interweaving at least one superelastic shape memory alloy wire diagonally in such a manner that upper and lower wires alternately cross over and under each other, thereby forming a plurality of diamond-shaped spaces in the hollow stent body, with an expanded part provided on at least one of opposite ends of the hollow stent body by increasing a diameter of the hollow stent body, wherein an anti-migration bent part may be provided on the expanded part or on both the expanded part and the hollow stent body by bending the wire such that the anti-migration bent part protrudes vertically and diagonally, thereby preventing migration of the stent in a lumen of a human body.

The present invention is advantageous in that the anti-migration stent of the present invention is provided with a hollow stent body formed by interweaving superelastic shape memory alloy wires diagonally such that the wires alternately cross over and under each other, with anti-migration bent parts formed on the hollow stent body by bending the wires such that the anti-migration bent parts protrude vertically and diagonally from the hollow stent body, so that when the stent of the present invention is implanted into a lumen of a human body to expand a lesion part therein, the stent is less likely to migrate from an inner surface of the lumen, and the desired implantation location is ensured. The present invention is also advantageous in that the anti-migration bent part of the present invention is prevented from pricking the inner surface of a lumen of a human body unlike a conventional anti-migration bent part that is slantingly bent back to prick the inner surface of the lumen of the human body when the anti-migration bent part is inserted into or removed from the lumen of the human body, and thus, it is easy to insert and remove the stent into and from the lumen, so that the inner surface of the lumen is prevented from being damaged by the anti-migration bent part.

In addition, the present invention is also advantageous in that the present invention provides excellent workability and productivity since the stent of the present invention is easily manufactured in such a manner that a hollow stent body is formed by interweaving superelastic shape memory alloy wires diagonally such that the wires alternately cross over and under each other, and that an anti-migration bent part vertically protrudes by placing and holding a rod underneath a wire of the hollow stent body and heating the stent body.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 is a front view illustrating an embodiment of a stent adopting the present invention;
FIG. 2 is an enlarged view of portion A of FIG. 1;
FIG. 3 is a view illustrating a process of forming an anti-migration bent part of the present invention;
FIG. 4 is an enlarged perspective view of an important part of FIG. 3;
FIGS. 5 and 6 are views illustrating various embodiments in which an anti-migration bent part is provided on an upper wire of the present invention;
FIG. 7 is a view illustrating an embodiment in which an anti-migration bent part is provided on a lower wire of the present invention;
FIG. 8 is a view illustrating an embodiment in which a large-sized anti-migration bent part is provided on an upper wire of the present invention;
FIGS. 9 and 10 are views illustrating embodiments in which a plurality of anti-migration bent parts are provided on an upper wire of the present invention;
FIG. 11 is a view illustrating an embodiment in which a silicone reinforcement part is formed on the inside of an anti-migration bent part of a wire of the present invention through coating;
FIGS. 12 and 13 are front views illustrating various embodiments of a stent adopting the present invention; and
FIGS. 14 to 16 are front views illustrating further embodiments of a stent adopting the present invention.

### DETAILED DESCRIPTION OF THE INVENTION

First, a method of manufacturing an anti-migration stent of the present invention will be described.

According to the present invention, a hollow stent body is produced by interweaving at least one superelastic shape memory alloy wire diagonally such that the wire alternately crosses over and under itself to form a plurality of diamond-shaped spaces in the hollow stent body, and a rod is placed and held underneath the wire of the hollow stent body, and the stent body is heated to form an anti-migration bent part on the wire, the anti-migration bent part protruding vertically to form an outline corresponding to a cross-sectional shape of the rod.

In this case, the size and number of anti-migration bent parts depend on the size of a diameter of the rod and the number thereof.

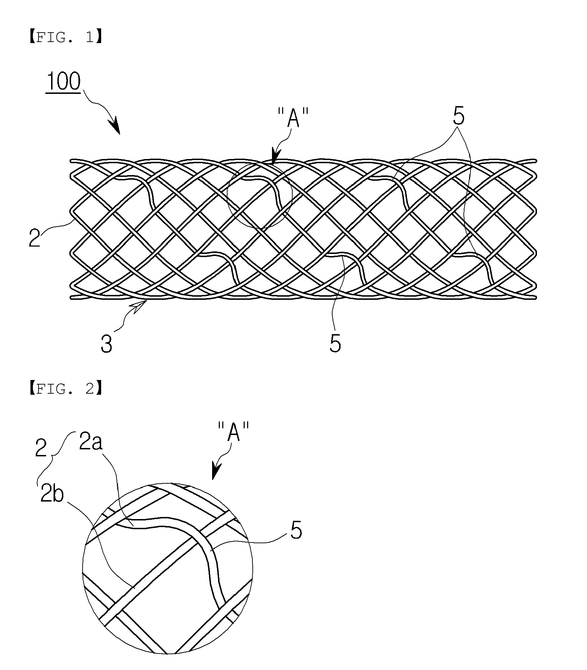

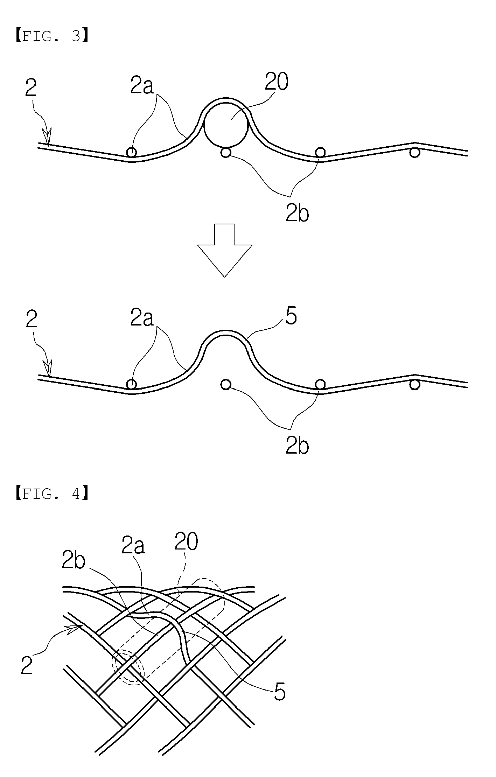

As illustrated in FIGS. 1 to 4, the present invention usually produces a hollow stent body using superelastic shape memory alloy wires. Thereafter, a rod is placed and tied underneath a wire of the hollow stent body, and the hollow stent body is heated to form an anti-migration bent part on the wire, the anti-migration bent part protruding vertically to form an outline corresponding to a cross-sectional shape of the rod. Accordingly, without respect to the structure of the stent, it is possible to easily manufacture the anti-migration bent part that can prevent migration of the stent installed in a lumen of a human body can be easily manufactured.

According to a method of the present invention, as illustrated FIGS. 1 to 10, an anti-migration stent 100 includes a hollow stent body 3 formed by interweaving at least one superelastic shape memory alloy wire 2 diagonally in such a manner that an upper wire 2a and a lower wire 2b alternately cross over and under each other, thereby forming a plurality of diamond-shaped spaces in the hollow stent body, and an anti-migration bent part 5 is provided on the hollow stent body 3 by bending the wire 2 such that the anti-migration bent part protrudes vertically and diagonally, thereby preventing migration of the stent in the lumen of the human body.

That is, the anti-migration bent part 5 is formed by bending an upper wire 2a of the wire 2 or a lower wire 2b of the wire 2 such that the anti-migration bent part protrudes vertically and diagonally, thereby preventing migration of the stent in the lumen of a human body.

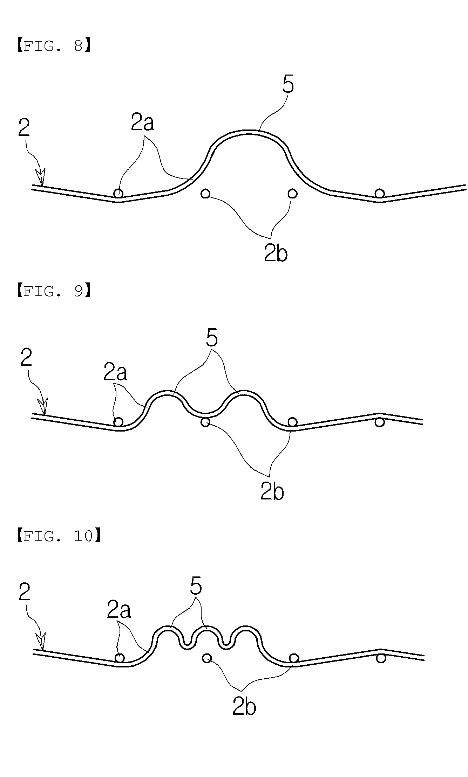

Particularly, the upper wire 2a is configured to pass over the lower wire 2b in such a manner that as illustrated in FIG. 8, the upper wire 2a passes over at least two lower wires 2b depending on the method of weaving the wire 2. Accordingly, it is possible to form the length of the upper wire 2a variously, and this allows the upper wire 2a to have various and sufficient lengths to allow a change in the size or number of the anti-migration bent part 5.

In addition, as illustrated in FIGS. 9 and 10, the anti-migration bent part 5 may include one or at least two anti-migration bent parts formed on an upper wire 2a or on a lower wire 2b, and the size or height of the anti-migration bent parts may be variously formed.

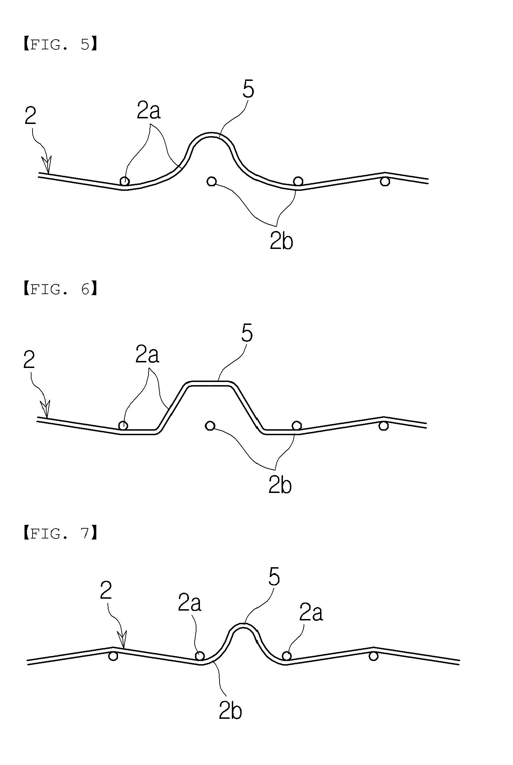

And as illustrated in FIG. 5, the anti-migration bent part 5 is preferred to be configured in a semicircular structure, but as illustrated in FIG. 6, the anti-migration bent part may be configured in a polygon or any other structure. Importantly, the anti-migration bent parts 5 are formed to protrude vertically, so it is easy to insert or remove the stent into or from a lumen of a human body, and in the course of doing this, the anti-migration bent part 5 may be variously formed not to prick and damage an inner surface of the lumen.

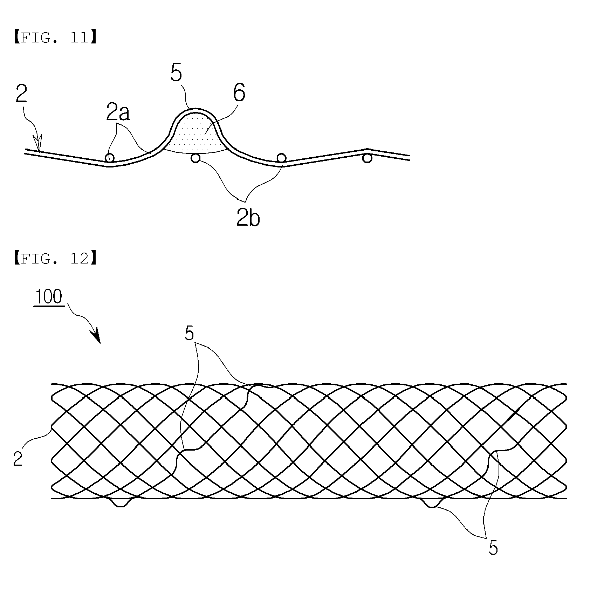

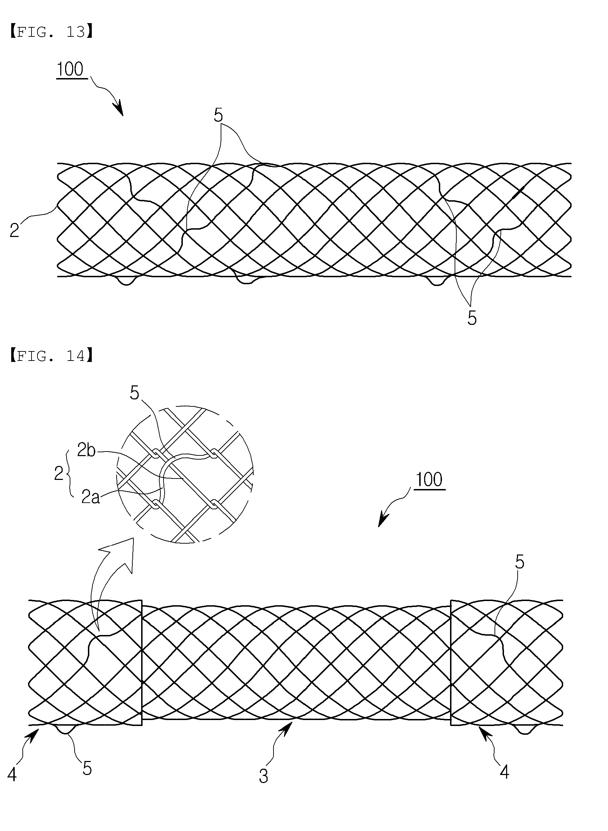

At least one anti-migration bent part 5 may be diagonally formed along a wire 2 of the hollow stent body 3. More particularly, as illustrated in FIG. 12, anti-migration bent parts 5 may be spirally formed in one diagonal direction, or as illustrated in FIG. 13, anti-migration bent parts may be spirally formed in two diagonal directions, or may be variously formed in complex shapes in two diagonal directions, and the anti-migration bent parts may also be formed in various numbers.

In addition, as illustrated in FIG. 11, a silicone reinforcement part 6 may be formed on an inside of the anti-migration bent part 5 through coating to increase the tension strength of the anti-migration bent part 5.

Furthermore, the hollow stent body 3 may be also provided with a cover (not shown) to close inner and outer parts of the hollow stent body, thereby preventing a lesion part from penetrating into an inner part of the hollow stent body 3. The cover may be made of a polyurethane, a silicone-urethane copolymer, a silicone, PTFE (Polytetrafluoroethylene), a polyamide, a polyester, Teflon, etc.

According to the present invention, when stenosis occurs due to tumors or other reasons in lumens of the human body, including digestive organs such as the duodenum, the biliary tract, and the esophagus, urinary organs such as the urethra, and respiratory organs such as the trachea, the human body cannot perform its normal function. Accordingly, an anti-migration stent 100 is inserted into the stenotic portion in a lumen of the human body to expand the stenotic portion so that the human body can function normally.

Particularly, the anti-migration stent 100 of the present invention includes a hollow stent body formed by interweaving a superelastic shape memory alloy wire diagonally in such a manner that an upper wire 2a and a lower wire 2b alternately cross over and under each other, thereby forming a plurality of diamond-shaped spaces in the hollow stent body 3, wherein an anti-migration bent part 5 is singularly or plurally provided on the hollow stent body 3 by bending the wire such that the anti-migration bent part protrudes vertically and diagonally, and the anti-migration stent is held by the anti-migration bent part 5 on an inner surface of a lumen of a human body, and thus migration of the stent is prevented at a lesion part in the lumen of the human body. Therefore, the anti-migration stent is stably and constantly held at the lesion part.

In addition, since the anti-migration stent 100 of the present invention is provided with a hollow stent body 3 having an anti-migration bent part formed by bending a wire 2 in such a manner that the anti-migration bent part 5 protrudes vertically and diagonally and in a semicircular or a polygonal structure, the anti-migration bent part 5 of the anti-migration stent can solve the problem of a conventional stent formed in a slanted shape that pricks an inner surface of a lumen of a human body when bent back upon insertion and removal from the lumen of the human body. Accordingly, the anti-migration stent is easy to insert into and remove from a lumen of a human body, thereby preventing the problem of pricking and damaging the inner surface of the lumen of the human body.

Additionally, according to the present invention, a silicone reinforcement part 6 may be formed on the inside of the anti-migration bent part 5 through coating to increase tension strength of the inner side and outer side of the anti-migration bent part 5, so the anti-migration stent can be more efficiently prevented from migrating from a lesion part at which the stent is installed.

Accordingly, an anti-migration stent 100 of the present invention can be stably held in a stenotic portion due to the anti-migration bent part 5 that can hold and prevent the anti-migration stent from migrating when inserting the stent into the stenotic portion to expand the stenotic portion. Therefore, the anti-migration stent of the present invention can increase the disease treatment effect, and an operation using the anti-migration stent of the present invention is easier than operation using a conventional stent when the stent is inserted into or removed from a lumen of a human body, thereby preventing the conventional problem of damaging an inner surface of the lumen of the human body and lessening the pain of a patient during an operation.

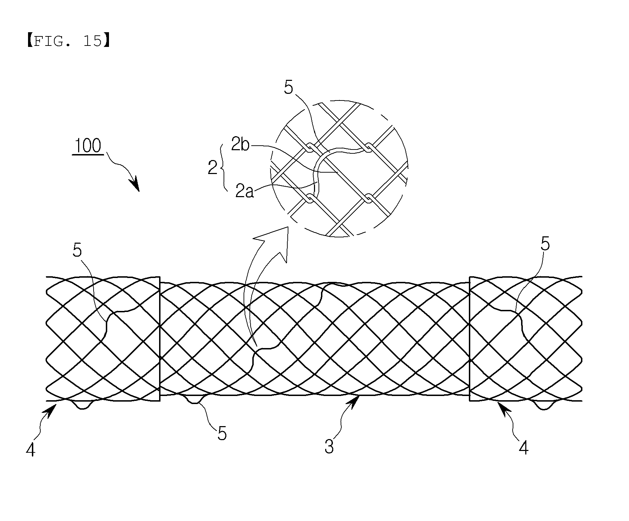

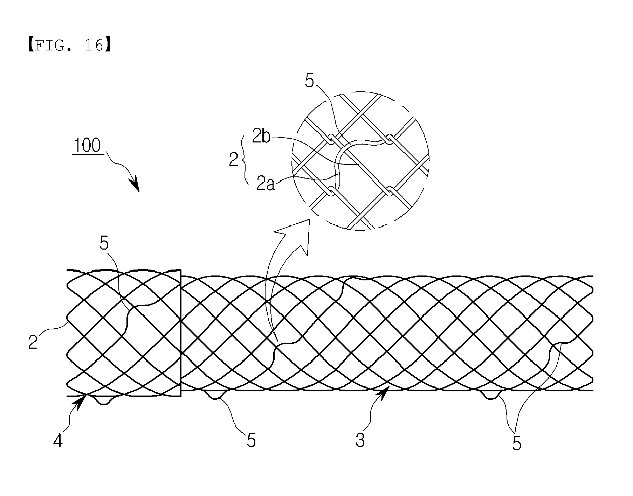

Other than the stent described above, as illustrated in FIGS. 14 to 16, the anti-migration stent 100 of the present invention may further include an expanded part 4 that is provided on at least one of opposite ends of the hollow stent body by increasing the diameter of the hollow stent body. In this embodiment, an anti-migration bent part 5 may be formed on the expanded part 4 of the stent or on both the hollow stent body 3 and the expanded part 4 by bending a wire 2 such that the anti-migration bent part 5 protrudes vertically and diagonally and can prevent migration of the stent in a lumen of a human body.

That is, in this embodiment, an anti-migration bent part 5 may be formed on the expanded part 4 or on both the hollow stent body 3 and the expanded part 4 by bending an upper wire 2a of a wire 2 or a lower wire 2b of the wire 2 such that the anti-migration bent part 5 protrudes vertically and diagonally, thereby preventing migration of the stent in a lumen of a human body.

In other words, in this embodiment, at least one anti-migration bent part 5 may be provided on an upper wire 2a and a lower wire 2b, and the size of the anti-migration bent part 5 can vary in the same manner as that described for the primary embodiment.

In addition, the anti-migration bent part 5 of this embodiment may be configured in a semicircular or polygonal structure. In other words, the anti-migration bent part 5 may be configured in various forms in such a manner that the anti-migration bent part 5 does not damage by pricking the inner surface of a lumen since it is easy to insert and remove the anti-migration bent part into and from the lumen. Further, a silicone reinforcement part 6 may be formed on the inside of the anti-migration bent part 5 through coating to increase the tension strength of the anti-migration bent part 5.

In this embodiment, at least one anti-migration bent part may be diagonally formed along a wire 2 of the hollow stent body 3. Here, the anti-migration bent part 5 may be spirally formed in one diagonal direction, or the anti-migration bent part 5 may be formed in two diagonal directions, or may be variously formed in complex shapes in two diagonal directions, and the anti-migration bent part 5 may also be formed in various numbers.

According to this embodiment, when a lumen of a human body has a stenotic portion due to tumors or other reasons and cannot perform a normal function, the anti-migration stent 5 is inserted into the stenotic portion to expand the stenotic portion in the same way as the primary embodiment, thereby enabling a normal function of the lumen.

Particularly, when compared to the primary embodiment, the anti-migration stent of the second embodiment further includes the expanded part 4 provided on at least one of opposite ends of the hollow stent body 3 by increasing the diameter of the hollow stent body, and the anti-migration stent includes an anti-migration bent part 5 formed on the expanded part 4 of the stent or on both the hollow stent body 3 and the expanded part 4 by bending a wire 2 such that the anti-migration bent part 5 protrudes vertically and diagonally, thereby preventing migration of the stent at a lesion part in the lumen of the human body by using the expanded part 4 provided on at least one of opposite ends of the hollow stent body 3, and the anti-migration bent part 5 formed on the expanded part 4 or on both the expanded part 4 and the hollow stent body 3 has an effect of preventing the migration of the stent, so that the stent can be stably held on the inner surface of a lesion of the lumen of the human body, thereby maximizing the effect of preventing the migration of the stent.

Furthermore, according to the second embodiment of the present invention, as in the primary embodiment, the anti-migration bent part 5 does not slantingly bend back to prick the inner surface of a lumen of a human body as a conventional anti-migration bent part does, so that it is easy to insert and remove the anti-migration stent having the anti-migration bent part into and from the lumen of the human body, thereby solving the problem of pricking and damaging the inner surface of the lumen of the human body.

Matters other than what were described above have the same operating effect as the primary embodiment, so further detailed description will be omitted.

The present invention is not limited to the application of the stent having the above-mentioned structure. The present invention may be applied to all stents capable of forming an anti-migration bent part 5 by bending a wire of a stent such that the anti-migration bent part protrudes vertically and diagonally, and those skilled in the art will appreciate that various modifications, additions and substitutions are possible, without departing from the scope and spirit of the invention as disclosed in the accompanying claims.

For the avoidance of doubt, the present application extends to the subject-matter described in the following numbered paragraphs (referred to as "Para" or "Paras"):
1. A method of manufacturing an anti-migration stent, the method comprising:
   producing a hollow stent body by interweaving at least one superelastic shape memory alloy wire diagonally such that the wire alternately crosses over and under itself to form a plurality of diamond-shaped spaces in the hollow stent body;
   placing and holding a rod underneath the wire of the hollow stent body; and
   heating the stent body to form an anti-migration bent part on the wire, the anti-migration bent part protruding vertically to form an outline corresponding to a cross-sectional shape of the rod.
2. An anti-migration stent constituted to prevent migration in a lumen of a human body, the anti-migration stent comprising:
   a hollow stent body formed by interweaving at least one superelastic shape memory alloy wire diagonally in such a manner that upper and lower wires alternately cross over and under each other, thereby forming a plurality of diamond-shaped spaces in the hollow stent body,
   wherein an anti-migration bent part is provided on the hollow stent body by bending the wire such that the anti-migration bent part protrudes vertically and diagonally, thereby preventing migration of the stent in the lumen of the human body.
3. The anti-migration stent of Para 2,
   wherein the anti-migration bent part is formed by bending the upper wire such that the upper wire protrudes upward vertically and diagonally from the hollow stent body.
4. The anti-migration stent of Para 3,
   wherein the upper wire is configured to pass over the lower wire in such a manner that the upper wire passes over at least two lower wires.
5. The anti-migration stent of Para 2,
   wherein the anti-migration bent part is formed by bending the lower wire such that the lower wire protrudes upward vertically and diagonally from the hollow stent body.
6. The anti-migration stent of Para 3 or 5,
   wherein the anti-migration bent part comprises at least two anti-migration bent parts that are continuously formed.
7. The anti-migration stent of Para 3 or 5,
   wherein the anti-migration bent part is configured in a semicircular structure.
8. The anti-migration stent of Para 3 or 5,
   wherein the anti-migration bent part is configured in a polygonal structure.
9. The anti-migration stent of Para 3 or 5,
   wherein a silicone reinforcement part is formed on an inside of the anti-migration bent part through coating to increase tension strength of the anti-migration bent part.
10. An anti-migration stent constituted to prevent migration in a lumen of a human body, the anti-migration stent comprising:
   a hollow stent body formed by interweaving at least one superelastic shape memory alloy wire diagonally in such a manner that upper and lower wires alternately cross up and down each other, thereby forming a plurality of diamond-shaped spaces in the hollow stent body, with an expanded part provided on at least one of opposite ends of the hollow stent body by increasing a diameter of the hollow stent body,
   wherein an anti-migration bent part is provided on the expanded part by bending the wire such that the anti-migration bent part protrudes vertically and diagonally, thereby preventing migration of the stent in a lumen of a human body.
11. The anti-migration stent of Para 10,
   the anti-migration bent part is formed by bending the upper wire such that the upper wire protrudes upward vertically and diagonally from the hollow stent body.
12. The anti-migration stent of Para 11,
   wherein the upper wire is configured to pass over the lower wire in such a manner that the upper wire passes over at least two lower wires.
13. The anti-migration stent of Para 10,
   wherein the anti-migration bent part is formed by bending the lower wire such that the lower wire protrudes upward vertically and diagonally from the hollow stent body.
14. The anti-migration stent of Para 11 or Para 13,
   wherein the anti-migration bent part comprises at least two anti-migration bent parts that are continuously formed.
15. The anti-migration stent of Para 11 or Para 13,
   wherein the anti-migration bent part is configured in a semicircular structure.
16. The anti-migration stent of Para 11 or Para 13,
   wherein the anti-migration bent part is configured in a polygonal structure.
17. The anti-migration stent of Para 11 or Para 13,
   wherein a silicone reinforcement part is formed on an inside of the anti-migration bent part through coating to increase tension strength of the anti-migration bent part.
18. An anti-migration stent constituted to prevent a migration in a lumen of a human body, the anti-migration stent comprising:
   a hollow stent body formed by interweaving at least one superelastic shape memory alloy wire diagonally in such a manner that upper and lower wires alternately cross over and under each other, thereby forming a plurality of diamond-shaped spaces in the hollow stent body, with an expanded part provided on at least one of opposite ends of the hollow stent body by increasing a diameter of the hollow stent body,
   wherein an anti-migration bent part is provided on each of the hollow stent body and the expanded part by bending the wire such that the anti-migration bent part protrudes vertically and diagonally, thereby preventing migration of the stent from the lumen of the human body.
19. The anti-migration stent of Para 18,
   wherein the anti-migration bent part is formed by bending the upper wire such that the upper wire protrudes upward vertically and diagonally from the hollow stent body.
20. The anti-migration stent of Para 19,
   wherein the upper wire is configured to pass over the lower wire in such a manner that the upper wire passes over at least two lower wires.
21. The anti-migration stent of Para 18,
   wherein the anti-migration bent part is formed by bending the lower wire such that the lower wire protrudes upward vertically and diagonally from the hollow stent body.
22. The anti-migration stent of Para 19 or Para 21,
   wherein the anti-migration bent part comprises at least two anti-migration bent parts that are continuously formed.
23. The anti-migration stent of Para 19 or Para 21,
   wherein the anti-migration bent part is configured in a semicircular structure.
24. The anti-migration stent of Para 19 or Para 21,
   wherein the anti-migration bent part is configured in a polygonal structure.
25. The anti-migration stent of Para 19 or Para 21,
   wherein a silicone reinforcement part is formed on an inside of the anti-migration bent part through coating to increase tension strength of the anti-migration bent part.

### <Description of the Reference Numerals in the Drawings>

2: Superelastic shape memory alloy (SMA) wire
2a: Upper wire
2b: Lower wire
3: Hollow stent body
4: Expanded part
5: Anti-migration bent part
6: Silicone reinforcement part

## Claims

1. A method of manufacturing an anti-migration stent, the method comprising:
producing a hollow stent body by interweaving at least one superelastic shape memory alloy wire diagonally such that the wire alternately crosses over and under itself to form a plurality of diamond-shaped spaces in the hollow stent body;
placing and holding a rod underneath the wire of the hollow stent body; and
heating the stent body to form an anti-migration bent part on the wire, the anti-migration bent part protruding vertically to form an outline corresponding to a cross-sectional shape of the rod.

2. An anti-migration stent constituted to prevent migration in a lumen of a human body, the anti-migration stent comprising:
a hollow stent body formed by interweaving at least one superelastic shape memory alloy wire diagonally in such a manner that upper and lower wires alternately cross over and under each other, thereby forming a plurality of diamond-shaped spaces in the hollow stent body,
wherein an anti-migration bent part is provided on the hollow stent body by bending the wire such that the anti-migration bent part protrudes vertically and diagonally, thereby preventing migration of the stent in the lumen of the human body.

3. An anti-migration stent constituted to prevent migration in a lumen of a human body, the anti-migration stent comprising:
a hollow stent body formed by interweaving at least one superelastic shape memory alloy wire diagonally in such a manner that upper and lower wires alternately cross up and down each other, thereby forming a plurality of diamond-shaped spaces in the hollow stent body, with an expanded part provided on at least one of opposite ends of the hollow stent body by increasing a diameter of the hollow stent body,
wherein an anti-migration bent part is provided on the expanded part by bending the wire such that the anti-migration bent part protrudes vertically and diagonally, thereby preventing migration of the stent in a lumen of a human body.

4. An anti-migration stent constituted to prevent a migration in a lumen of a human body, the anti-migration stent comprising:
a hollow stent body formed by interweaving at least one superelastic shape memory alloy wire diagonally in such a manner that upper and lower wires alternately cross over and under each other, thereby forming a plurality of diamond-shaped spaces in the hollow stent body, with an expanded part provided on at least one of opposite ends of the hollow stent body by increasing a diameter of the hollow stent body,
wherein an anti-migration bent part is provided on each of the hollow stent body and the expanded part by bending the wire such that the anti-migration bent part protrudes vertically and diagonally, thereby preventing migration of the stent from the lumen of the human body.

5. The anti-migration stent of any of claims 2-4,
wherein the anti-migration bent part is formed by bending the upper wire such that the upper wire protrudes upward vertically and diagonally from the hollow stent body.

6. The anti-migration stent of any of claims 2-5,
wherein the upper wire is configured to pass over the lower wire in such a manner that the upper wire passes over at least two lower wires.

7. The anti-migration stent of any of claims 2-6,
wherein the anti-migration bent part is formed by bending the lower wire such that the lower wire protrudes upward vertically and diagonally from the hollow stent body.

8. The anti-migration stent of any of claims 2-7,
wherein the anti-migration bent part comprises at least two anti-migration bent parts that are continuously formed.

9. The anti-migration stent of any of claims 2-8,
wherein the anti-migration bent part is configured in a semicircular structure.

10. The anti-migration stent of any of claims 2-9,
wherein the anti-migration bent part is configured in a polygonal structure.

11. The anti-migration stent of any of claims 2-10,
wherein a silicone reinforcement part is formed on an inside of the anti-migration bent part through coating to increase tension strength of the anti-migration bent part.
